# FASCICULE DE BREVET EUROPEEN

(11) **EP 2 099 847 B1**
(45) Date de publication et mention de la délivrance du brevet: **10.09.2014**
(21) Numéro de dépôt: 07822686.7
(22) Date de dépôt: 16.11.2007
(51) Int. Cl.: C08J 3/05, C08J 3/28, C08L 1/28, A61L 27/26, A61L 27/48, A61L 27/52, A61K 47/38, A61K 47/48

(54) **HYDROGEL ET SES APPLICATIONS BIOMÉDICALES**
HYDROGEL UND BIOMEDIZINISCHE ANWENDUNGEN DAVON
HYDROGEL AND BIOMEDICAL APPLICATIONS THEREOF

(30) Priorité: 17.11.2006 FR 0610064
(43) Date de publication de la demande: 16.09.2009
(73) Titulaire: Biomatlante, 44360 Vigneux de Bretagne (FR)
(72) Inventeur: BOURGES, Xavier, 01140 Mogneneins (FR); BAROTH, Serge, 44100 Nantes (FR)
(74) Mandataire: Regimbeau
(86) Numéro de dépôt international: PCT/EP2007/062470
(87) Numéro de publication internationale: WO 2008/059058

(56) Documents cités:
- WO-A-01/30407
- WO-A2-2007/079053
- FR-A1- 2 715 853
- GB-A- 2 229 443
- US-A- 5 545 442
- US-A1- 2005 003 009
- US-B1- 6 638 538
- WACH R A ET AL: "Radiation crosslinking of methylcellulose and hydroxyethylcellulose in concentrated aqueous solutions" NUCLEAR INSTRUMENTS & METHODS IN PHYSICS RESEARCH, SECTION - B: BEAM INTERACTIONS WITH MATERIALS AND ATOMS, ELSEVIER, AMSTERDAM, NL, vol. 211, no. 4, décembre 2003 (2003-12), pages 533-544, XP004470037 ISSN: 0168-583X cité dans la demande
- DATABASE WPI Week 200439 Derwent Publications Ltd., London, GB; AN 2004-413852 XP002439821 & JP 2004 059926 A (JAPAN ATOMIC ENERGY RES INST) 26 février 2004 (2004-02-26)
- DATABASE WPI Week 200528 Derwent Publications Ltd., London, GB; AN 2005-266750 XP002439822 & JP 2005 082800 A (UNIV KINKI) 31 mars 2005 (2005-03-31)
- DATABASE WPI Week 200431 Derwent Publications Ltd., London, GB; AN 2004-333969 XP002439823 & JP 2004 043543 A (JAPAN ATOMIC ENERGY RES INST) 12 février 2004 (2004-02-12)
- DATABASE WPI Week 200630 Derwent Publications Ltd., London, GB; AN 2006-288269 XP002439824 & JP 2006 102227 A (JAPAN ATOMIC ENERGY RES INST) 20 avril 2006 (2006-04-20)
- DATABASE WPI Week 200132 Derwent Publications Ltd., London, GB; AN 2001-303511 XP002439825 & JP 2001 002703 A (DAICEL CHEM IND LTD) 9 janvier 2001 (2001-01-09)

## Description

La présente invention concerne de nouveaux hydrogels homogènes injectables réticulés obtenus à partir du mélange de 2 ou 3 polymères hydrosolubles.

La réticulation des polymères et la stérilité du matériau sont obtenus simultanément au moyen d'irradiation β ou γ. La concentration en polymères et la dose de radiation doivent être ajustées précisément, en fonction des propriétés physiques de chaque matière première polymérique. Les hydrogels selon la présente invention conviennent à des applications bio-médicales.

Les polymères hydrosolubles et les hydrogels sont largement utilisés dans le domaine médical, notamment en tant que biomatériaux implantables. Ils peuvent être utilisés seuls ou en association avec d'autres phases, comme des particules (métalliques, minérales ou organiques) et/ou des substances actives.

Tous les hydrogels ont une structure similaire de type gélatine, bien que leurs propriétés physiques peuvent varier grandement, en fonction de leurs réseaux tridimensionnels, de leurs concentrations, de leur taux de gonflement et de leur caractère hydrophile.

Deux catégories générales d'hydrogels peuvent être définies : (a) les gels physiques (pseudo gels), dans lesquels les chaînes sont interconnectées par des forces électrostatiques, des liaisons hydrogènes, des interactions hydrophobes ou des enchevêtrements de chaînes. De tels gels sont non permanents et peuvent être habituellement convertis en solutions polymériques par chauffage. (b) Les gels chimiques (vrais, permanents), dans lesquels des liaisons covalentes relient les chaînes.

L'avantage principal de l'utilisation d'hydrogels pour la préparation de biomatériaux réside dans la possibilité d'obtenir de nouveaux tissus présentant un volume et une cohésion suffisants, tout en étant inertes et/ou biodégradables. Les hydrogels peuvent aussi être utilisés dans des traitements de surface, par exemple pour obtenir des surfaces biomimétiques, des pansements ou dans des systèmes d'administration de médicaments.

Ces hydrogels sont généralement obtenus à partir de polymères hydrosolubles liquides ou visqueux. Comme pour les hydrogels, ces polymères hydrosolubles liquides ou visqueux sont généralement utilisés pour obtenir un certain volume de tissus ou pour véhiculer des particules solides, en fonction du site d'implantation. Dans ces solutions, l'eau comble l'espace entre les chaînes polymériques. Les polymères hydrosolubles disparaissent ensuite, en un temps variant selon l'irrigation de la zone opérée par les liquides corporels ainsi que de la biodégradabilité du gel lui-même.

En chirurgie oculaire, le rôle principal des polymères hydrosolubles est d'augmenter le volume du segment intérieur de la chambre oculaire. Le hyaluronate de sodium et l'hydroxypropylméthylcellulose sont deux exemples de polymères hydrosolubles utilisés commercialement pour de telles applications.

Les polymères hydrosolubles trouvent une autre application avec l'injection par arthroscopie de hyaluronate de sodium. Le brevet américain n° US 5,470,578 décrit une composition antirhumatismale basée sur un glycosaminoglycane lié à un lipide. Ce produit, dont la durée de vie est d'environ 6 à 9 mois, permet une lubrification articulaire.

La demande de brevet WO 95/21634 décrit un substitut osseux original développé en 1992. Il est constitué d'hydroxypropylméthylcellulose (HPMC), associée à des granules de phosphate de calcium biphasique, le rôle principal du polymère hydrosoluble étant de véhiculer les granules durant l'injection. Plusieurs produits commerciaux sont ensuite apparus sur le même principe. L'un deux, décrit dans la demande de brevet européen EP 1 080 737, la carboxyméthylcellulose joue le même rôle que l'HPMC.

Les polymères hydrosolubles trouvent aussi des applications en chirurgie esthétique. De nombreux produits existent dans ce domaine et sont basés sur le hyaluronate de sodium et ses dérivés, la carboxyméthylcellulose, la polyacrylamide, la polyvinylpyrolidone, etc...

Le développement de nouveaux biomatériaux va aujourd'hui dans la direction de matériaux composites. Le mélange de différentes sortes de polymères hydrosolubles permet d'obtenir des hydrogels présentant de nouvelles propriétés rhéologiques (thixotropie, plasticité, caractère hydrophile...) De façon intéressante, la biocompatibilité du mélange peut aussi être améliorée par rapport à celle de chacun des polymères de départ.

Des réactions chimiques entre des polymères hydrosolubles peuvent être conduites afin d'obtenir des hydrogels par voie chimique, mais les techniques de radiation peuvent être aussi mises en oeuvre pour l'obtention d'hydrogels. Avantageusement, les techniques de radiation permettent de stériliser en même temps le produit final. Les radiations créent des liaisons covalentes entre les polymères ; elles modifient le poids moléculaire, le caractère hydrophile et les propriétés mécaniques des polymères de départ, soit par irradiation directe des polymères ou en greffant d'autres motifs polymériques sur les chaînes. Un autre avantage de cette technique est lié au fait qu'aucun initiateur de polymérisation toxique ou catalyseur non biocompatible n'est utilisé. Malheureusement, le fait que beaucoup de modifications incontrôlées puissent se produire lors de l'irradiation, en particulier avec des polysaccharides, reste un inconvénient majeur de cette technique. Le fait principal de l'irradiation sur les polysaccharides est la dégradation ; la seconde étant la réticulation du polymère, en fonction de sa structure chimique. Les deux processus peuvent avoir lieu simultanément et leur rendement détermine la structure du produit final.

De façon intéressante, des polymères hydrosolubles différents ne réagissent pas de la même manière à l'irradiation. La carboxyméthylcellulose (CMC) est plus sensible à l'irradiation que la méthylcellulose (MC) ou l'hydroxypropylméthylcellulose (HPMC) en raison du caractère ionique de la carboxyméthylcellulose.

La dose de radiation et la concentration jouent aussi un rôle significatif. En général, pour des hautes concentrations en polymères, la réticulation se produit facilement quand la dose de radiation est optimale, les interactions entre les chaînes polymériques étant plus nombreuses que dans les systèmes dilués. A l'opposé, si la concentration en polymères est insuffisante, le phénomène de dégradation prend une proportion plus importante que la réticulation et la solution polymérique devient finalement totalement liquide.

Beaucoup de scientifiques ont étudié l'effet de l'irradiation sur la réticulation des polymères. En particulier, l'équipe du Takasaki Radiation Chemistry Research Establishment, dirigée par Kaetsu, ainsi que Hoffman et ses collègues du Center of Bioengineering à l'Université de Washington, ont déclenché un intérêt grandissant pour la formation de biomatériaux au moyen de radiations, et pour l'étude du phénomène de réticulation.

Récemment, Nasagawa et Yoshii (Carbohydrate Polymers 2004, 58, 109-113 *;* Nuclear Instruments and Methods in Physics Research Section B : Beam Interactions with Materials and Atoms 2003, 208, 320-324) ont synthétisé différents hydrogels à partir de dérivés d'amidon, en utilisant des radiations et sans l'aide d'additif. Ils ont découvert que des concentrations élevées de polymères en solution aqueuse (les conditions dites « paste-like ») favorisent la réticulation. A l'opposé, à de faibles concentrations, le phénomène de réticulation ne se produit pas et c'est principalement une dégradation qui est observée sous forme d'une chute de la viscosité. La quantité de polymères n'était pas suffisante dans ce cas pour obtenir la réticulation.

Wach et al. (Nuclear Instruments and Methods in Physics Research section B: Beam Interactions with Materials and Atoms 2003, 211, 533-544) ont décrit le premier hydrogel réticulé comprenant deux éthers de cellulose, la méthylcellulose et l'hydroxyéthylcellulose. Dans ce cas encore, les concentrations en polymères étaient très élevées.

Pekel et al. (Carbohydrate Polymers 2004, 55, 139-147) ont étudié la réticulation par irradiation de l'hydroxypropylméthylcellulose seule en solution. L'irradiation de l'HPMC en solutions aqueuses à des concentrations raisonnables (« paste-like condition ») a permis d'obtenir la formation de gel. Le pourcentage de gel a été augmenté par l'utilisation de grandes quantités de radiations.

L'abrégé de JP 2004 059926 (document XP 002439421) décrit des compositions capables de former un hydrogel comprenant (en pourcentage massique) de l'amidon (30), de la polyvinyl pyrrolidone (20), du polyvinylalcohol (15), de la cellulose hydrosoluble (10) et de l'eau. La concentration de ces éléments dans la solution aqueuse n'est pas précisée. En outre, le ratio en poids entre la cellulose hydrosoluble et les polymères hydrosolubles est inférieur à 1. Enfin, il n'est pas indiqué si la composition est irradiée.

Le brevet US 6,638,538 décrit des hydrogels comprenant de l'acide hyaluronique et un autre polymère, en particulier la CMC, formés par des cycles répétés de congélation/décongélation de solutions aqueuses acides de ces polymères. Ces hydrogels sont irradiés après la réticulation pour stériliser l'hydrogel destiné à être implanté.

Le document WO 2007/079053 décrit une composition de substitut osseux comprenant un hydrogel d'un polymère hydrosoluble dérivé de la cellulose et des particules solides. L'hydrogel est réticulé par irradiation UV ou lumière visible en utilisant des initiateurs de la polymérisation qui génèrent des radicaux libres. La composition de WO 2007/079053 implique la présence obligatoire d'un accélérateur de la polymérisation UV. Ce système de polymérisation est activé par la lumière pour polymériser le gel véhicule. Le système de polymérisation comprend un initiateur de la photo polymérisation qui génère des radicaux libres quand il est irradié par les ultraviolets ou la lumière visible. De tels initiateurs de polymérisation sont par exemple l'éosine, l'acridine, la thiazine, la xanthine et la phénazine. La présence du système de polymérisation est essentielle à la polymérisation du gel décrit dans WO 2007/079053.

Diverses techniques ont été mises à disposition afin de décrire un hydrogel irradié. Un des outils principaux est l'analyse sol-gel, qui repose sur la mesure gravimétrique de la solution et de la fraction de gel après irradiation. La méthode peut facilement être mise en oeuvre et donne accès au rendement de la réaction de la réticulation, ainsi qu'au taux de dégradation et de scission des chaînes polymériques.

Les calculs sont d'habitude basés sur l'équation Charlesby-Pinner (Atomic Radiation and Polymers; Pergamon Press, Oxford (1960)), dont une version plus générale a été développée par la suite (Radiat. Phys. Chem, 1998, 51, 13 *;* Radial. Phys. Chem. 1991, 37, 499). Ainsi que l'a décrit Rosiak, « le réseau de polymères et la structure du gel ont un comportement totalement différent des molécules de départ ou des molécules partiellement réticulées; si ils sont flexibles, ils peuvent avoir des propriétés de caoutchouc, ils peuvent gonfler. La dose requise pour atteindre le point de gel est Dg et la densité de réticulation due à une dose plus forte D est souvent exprimée en termes de coefficient δ relié à D et Dg ; δ = D/Dg. En continuant l'irradiation (δ > 1) la fraction de polymères liés entre eux en un réseau ou en gel augmente (fraction de gel - g), alors que la fraction soluble restante diminue; s + g = 1. La relation entre la fraction soluble s et la dose D dépend de la distribution initiale des poids moléculaires moyens. La théoric de l'interaction d'une radiation ionisante avec la matière suppose que cette réticulation ait lieu au hasard et proportionnellement à la dose. »

Un avantage supplémentaire lié à l'utilisation de radiations est qu'elle permette d'obtenir simultanément un hydrogel ainsi que la stérilité du produit, alors que des procédés aseptiques ou des étapes additionnelles de stérilisation sont traditionnellement utilisées à cette fin (vapeur, plasma, oxyde d'éthylène, filtration...).

On entend par stérilité l'état d'un matériau dépourvu de microorganismes.

Selon la nature du matériau médical, l'homme de l'art s'efforce d'obtenir la stérilité par la technique la plus adaptée et la moins dégradante pour le produit. Les procédés aseptiques et la filtration sont les deux méthodes qui induisent le moins de modifications des polymères hydrosolubles. A l'opposé, il n'est pas possible de stériliser les hydrogels et les polymères hydrosolubles en utilisant l'oxyde d'éthylène sans modification car le gaz réagit rapidement avec l'eau pour former du glycol.

Le brevet US 4,871,490 décrit un hydrogel utilisé en tant que pansement, obtenu et stérilisé au moyen de radiations sous la forme de fines couches gonflées constituées de polyvinylpyrolidone, de polyéthylène glycol et agar.

Les polymères dérivés de la cellulose présentent des propriétés intéressantes pour des applications biomédicales. Ils sont solubles dans l'eau, chimiquement stables et non toxiques. Malheureusement, comme mentionné ci-dessus, ils sont plus sensibles aux radiations que les autres types de polymères.

La technique de l'irradiation présente l'avantage séduisant d'obtenir la stérilité en même temps que la formation du gel. Mais les « paste-like conditions » requises, lorsque des polymères dérivés de la cellulose sont employés, posent problème lorsque l'hydrogel souhaité est destiné à l'injection.

Les auteurs de la présente invention ont résolu ce problème et trouvé de façon surprenante qu'un hydrogel stérile et injectable, basé un polymère hydrosoluble dérivé de la cellulose, pouvait être obtenu grâce à cette technique, à des concentrations très basses.

Les inventeurs ont également trouvé que la réticulation est plus efficace quand le polymère dérivé de la cellulose est en présence d'autres polymères qui sont moins sensibles aux radiations.

De plus, les inventeurs ont trouvé que la quantité et la concentration en polymères devaient être finement ajustées pour obtenir les propriétés désirées après l'irradiation, par exemple un produit liquide, visqueux ou sous la forme d'un hydrogel.

Les inventeurs ont aussi trouvé que la dose de radiation doit être finement ajustée afin de transformer les polymères et, dans le même temps, stériliser le produit final selon la norme.

Plus précisément, l'invention concerne un procédé de préparation d'un hydrogel homogène stérile réticulé injectable, caractérisé en ce qu'il comprend les étapes successives suivantes :
(a) préparer une solution aqueuse comprenant un polymère dérivé de la cellulose et au moins un second polymère hydrosoluble, la concentration totale en polymère étant comprise en poids entre 0,5% et 5%, de préférence comprise entre 1% et 4%, et plus préférentiellement encore comprise entre 1,5% et 3% du poids total, le ratio en poids de polymère dérivé de la cellulose second polymère hydrosoluble étant compris entre 70/30 et 85/15, préférentiellement égal à 75/25.
(b) éventuellement ajouter des particules solides ;
(c) verser le mélange liquide résultant, et contenant les éventuelles particules solides, dans un récipient et fermer ledit récipient au moyen d'un système imperméable à l'eau et au gaz ;
(d) exposer le récipient contenant le liquide et les éventuelles particules solides à une dose de radiations β ou γ comprise entre 5 et 50 kGy, de préférence comprise entre 20 et 30 kGy, et plus préférentiellement encore environ 25 kGy.

On entend par radiation β, des rayons β consistant en l'émission de particules β, les particules β étant des particules d'électrons ou de positrons.

On entend par radiation γ, des rayons γ qui consistent en des ondes électromagnétiques ayant une énergie supérieure ou égale à 10 kcV. On cite à titre d'exemple de radiations γ et de façon non limitative les rayons-X.

On entend par polymère hydrosoluble dérivé de la cellulose, un polymère de cellulose dans lequel les groupements hydroxyles ont été remplacés par divers substituants. On cite à titre d'exemple de polymère dérivé de la cellulose et de façon non limitative l'hydroxypropylméthylcellulose (HPMC), la méthylcellulose (MC), l'hydroxyéthylcellulose (HEC), l'hydroxypropylcellulose (HPC) et la carboxyméhylcellulose (CMC).

On entend par polymère hydrosoluble un polymère soluble dans l'eau qui n'est pas toxique pour les mammifères et en particulier pour les humains.

Les hydrogels selon la présente invention peuvent être utilisés en ingénierie tissulaire. Ils peuvent être injectés à l'aide d'une seringue ou d'une canule.

Le mélange aqueux peut aussi remplir un moule et ensuite être exposé aux radiations.

Le polymère hydrosoluble dérivé de la cellulose selon l'invention peut être choisi dans le groupe comprenant l'hydroxypropylméthylcellulose (HPMC) et la méthylcellulose (MC).

Selon un mode de réalisation avantageux de l'invention, le polymère hydrosoluble dérivé de la cellulose est l'HPMC.

Le second polymère hydrosoluble peut être choisi dans le groupe comprenant la carboxyméthylcellulose (CMC), les glycosaminoglycanes (GAG), la polyvinylpyrolidone (PVP), le polyéthylèneglycol (PEG), le polyvinylalcool (PVA) et l'alginate de sodium.

Selon un mode de réalisation avantageux de l'invention, le second polymère hydrosoluble est la CMC.

Avantageusement, le glycosaminoglycane (GAG) selon l'invention peut être choisi dans le groupe comprenant le hyaluronate de sodium, l'acide hyaluronique, le sulfate de dermatan, le sulfate de kératine, le sulfate d'héparan, le sulfate de chondroïtine et le chitosan.

Selon un mode de réalisation avantageux de l'invention, le glycosaminoglycane est le hyaluronate de sodium.

Selon un autre mode de réalisation avantageux de l'invention, le mélange aqueux comprend l'HPMC et la CMC.

Dans un autre mode de réalisation avantageux de l'invention, le mélange aqueux comprend l'HPMC et le hyaluronate de sodium.

Avantageusement, dans le procédé selon l'invention :
- Le polymère hydrosoluble dérivé de la cellulose est l'hydroxypropylméthylcellulose à une concentration en poids comprise entre 0,5% et 2,5%, avantageusement entre 1% et 2%, et de façon encore plus avantageuse environ 1,5% du poids total ; et
- Le second polymère hydrosoluble est choisi dans le groupe comprenant la carboxyméthylcellulose à une concentration en poids comprise entre 0,1% et 1%, avantageusement entre 0,25% et 0,7%, et plus avantageusement encore environ 0,5% du poids total ou le hyaluronate de sodium à une concentration en poids comprise entre 0,1% et 1%, avantageusement comprise entre 0,25% et 0,7%, et plus avantageusement encore environ 0,5% du poids total.

L'HPMC et la MC sont des polymères non ioniques au contraire de la CMC. Au cours de l'irradiation β, le polymère nouvellement formé à partir de HPMC et CMC est ionique. En fonction de la concentration en polymères, le produit formé peut être liquide, visqueux ou sous la forme d'un hydrogel. Dans notre cas, un hydrogel homogène est recherché. Le produit final est un hydrogel lorsque la réticulation a été très efficace.

Le mélange aqueux de polymères hydrosolubles comprend en outre de façon avantageuse au moins un polymère biocompatible.

Le polymère biocompatible peut être choisis dans le groupe comprenant l'acide polylactique, les copolymères d'acide polylactique et d'acide polyglycolique, la poly ε caprolactone, les polyhydroxybutyrates, les polyhydroxyvalérates, les copolymères de polyhydroxybutyrates et polyhydroxyvalérates, les polyuréthanes, la cellulose, le polyéthylène, les polycarbonates, le polyméthylméthacrylate, les silicones, les polyamides et l'acide polyglycolique.

Les particules solides selon l'invention peuvent être choisies dans le groupe comprenant le phosphate de calcium biphasique (BCP), le phosphate tricalcique β (Ca₃(PO₄)₂), l'hydroxyapatite (Ca₁₀(PO₄)₆(OH)₂), le sulfate de calcium, le zirconite d'alumine, le nasicon, les bioverres, les vitrocéramiques, les nanoparticules, les composés radio-opaques ainsi que les mélanges comprenant plusieurs de ces composés.

Avantageusement, les particules solides sont des granules de BCP.

Le BCP est un mélange constitué d'hydroxyapatite et de phosphate tricalcique β.

Avantageusement, le ratio hydroxyapatite/phosphate tricalcique β est compris entre 10/90 et 90/10, avantageusement compris entre 20/80 et 20/80, avantageusement environ 60/40.

Le BCP est connu comme étant un ostéoconducteur et est largement utilisé en tant que substitut osseux. Dans le substitut osseux selon l'invention, l'hydrogel joue le rôle d'un espaceur entre les granules afin de permettre la colonisation cellulaire sur la totalité du produit durant la constitution osseuse.

La quantité de particules solides dans l'hydrogel est avantageusement comprise en poids entre 15% et 75%, avantageusement comprise entre 30% et 60% du poids total.

Selon un mode avantageux de l'invention, le poids des particules solides dans l'hydrogel représente environ 55 % du poids total.

Avantageusement le polymère dérivé de la cellulose est l'HPMC et le polymère hydrosoluble est la CMC ou un GAG, ledit GAG étant avantageusement le hyaluronate de sodium et lesdites particules solides étant avantageusement des granules de BCP.

Dans un mode de réalisation particulièrement avantageux de l'invention, le mélange aqueux comprend de l'HPMC, de la CMC et des granules de BCP.

Selon un mode de réalisation avantageux de l'invention, le mélange contient en poids :
- 45% d'un mélange aqueux d'hydroxypropylméthylcellulose et de carboxyméthylcellulose ;
- 44% de granules de phosphate de calcium biphasique de diamètre compris entre 80 et 200 µm dans lequel le ratio hydroxyapatite/phosphate tricalcique β est 60/40 ; et
- 11% de granules de phosphate de calcium biphasique de diamètre compris entre 0,5 et 1,0 mm dans lequel le ratio hydroxyapatite/phosphate tricalcique β est 60/40.

Selon un autre mode de réalisation avantageux de l'invention, le mélange aqueux contient en poids :
- 45% d'un mélange aqueux d'hydroxypropylméthylcellulose à une concentration en poids de 1,5% et de carboxyméthylcellulose à une concentration en poids de 0,5% ;
- 44% de granules de phosphate de calcium biphasique de diamètre compris entre 80 et 200 µm dans lequel le ratio hydroxyapatite/phosphate tricalcique β est 60/40 ; et
- 11% de granules de phosphate de calcium biphasique de diamètre compris entre 0,5 et 1,0 mm dans lequel le ratio hydroxyapatite/phosphate tricalcique β est 60/40.

Des substances actives peuvent être ajoutées aux hydrogels de l'invention après irradiation.

On entend par substances actives des molécules présentant une activité pharmaceutique ou cosmétique.

La substance active peut être choisie de façon non limitative dans le groupe comprenant les antibiotiques, les facteurs de croissance, les hormones, les peptides, les anti-ostéoporose et les anti-tumoraux.

On cite à titre d'exemple de récipients convenant pour réaliser le procédé selon l'invention, et de façon non limitative, les seringues, les canules ou les moules. Ces récipients peuvent être en verre, en inox ou en silicone.

Le procédé selon l'invention peut comprendre une étape optionnelle (e) consistant à déshydrater l'hydrogel obtenu au terme de l'étape (d).

Selon la présente invention, le ratio en poids polymère dérivé de la cellulose/second polymère hydrosoluble est compris entre 70/30 et 85/15, plus avantageusement encore égal à environ 75/25.

L'invention a également pour objet les hydrogels susceptibles d'être obtenus selon le procédé décrit précédemment.

Les hydrogels selon l'invention ne comprenant pas de particules solides peuvent être utilisés, de façon non limitative, comme pansement humidifiant pour brûlures et escarres, ou sous la forme d'une membrane pour réduire les adhésions post-chirurgicales après une laparotomie abdominale ou pelvienne.

Les hydrogels selon l'invention ne comprenant pas de particules solides peuvent aussi être utilisés pour combler des rides en chirurgie esthétique ou afin de redonner du volume aux tissus mous.

Les hydrogels selon l'invention peuvent en outre comprendre des particules solides afin d'être utilisés en temps que substituts osseux.

Plusieurs substituts osseux synthétiques de résorption sont déjà disponibles sous forme solide, sous forme d'un ciment ou d'un mastique, selon l'usage auquel ils sont destinés, selon le site d'implantation, ainsi que selon la cinétique de repousse osseuse.

L'avantage principal des substituts osseux synthétiques sur les greffons naturels est lié à leur innocuité, puisqu'ils sont dépourvus de virus, prions ou bactéries. Ainsi, si l'auto-greffon n'est pas disponible en quantité suffisante, le substitut osseux peut être ajouté en supplément. Ils ont aussi démontré leur efficacité en terme d'ostéoconduction en comparaison aux auto-greffons (les étalons de référence) et peuvent maintenant être utilisés seuls.

Les substituts osseux selon l'invention peuvent être utilisés en vertébroplastie, à la place des ciments de type polyméthylméthacrylate (PMMA) communément utilisés. Ces derniers sont biocompatibles, mais ne permettent pas une résorption osseuse naturelle. En effet, la dislocation des vertèbres voisines de celles opérées est souvent observée après quelques années en raison de la différence rhéologique entre l'intérieur des vertèbres naturelles et les vertèbres remplies par un ciment PMMA. Ce problème est évité par l'utilisation d'un matériau de résorption selon la présente invention.

Selon la présente invention, le substitut osseux peut aussi être utilisé en chirurgie maxillo-faciale, en particulier pour soigner les défauts osseux, pour combler les sinus, pour le comblement d'alvéoles dentaires afin de maintenir la hauteur de crête gingivale, en reconstruction de la cavité mastoïdienne.

Le substitut osseux selon l'invention peut de plus être utilisé en chirurgie orthopédique, en chirurgie de comblement d'une cavité tumorale, pour soigner des fractures avec défauts osseux, contre la pseudo-arthrose avec ou sans défaut osseux.

Lors de la résorption osseuse mettant en oeuvre les substituts osseux selon l'invention, les cellules osseuses résorbent et remplacent le biomatériau avec de l'os nouvellement formé présentant un taux de minéralisation élevé. Des cycles résorption/apposition se produisent comme dans l'os traditionnel, et le volume osseux augmente progressivement au dépend du gel.

Le BCP associe la stabilité de l'hydroxyapatite, qui agit comme un bon support d'adhésion pour les cellules ostéogéniques, aux bonnes propriétés de relargage d'ions du phosphate tricalcique β qui promeut la résorption cellulaire.

Le substitut osseux selon l'invention peut de plus être utilisé dans des cages de fusion en arthrodèse vertébrale (fusion vertébrale). La cage peut être remplie du mélange polymérique et durant l'étape de stérilisation (par exposition aux radiations), le gel est inclut dans la cage.

L'invention a également pour but l'utilisation d'un hydrogel tel que défini précédemment en tant que médicament.

L'invention a également pour but l'utilisation d'un hydrogel tel que défini précédemment en tant que substitut osseux.

L'invention a également pour but l'utilisation d'un hydrogel tel que défini précédemment pour la fabrication d'un médicament destiné à traiter les défauts osseux, ou d'un médicament destiné à combler les sinus, ou d'un médicament destiné à combler les d'alvéoles dentaires afin de maintenir la hauteur de crête gingivale, ou d'un médicament destiné à reconstruire la cavité mastoïdienne.

L'invention a encore pour but l'utilisation d'un hydrogel tel que défini précédemment pour la fabrication d'un médicament destiné à traiter les cavités tumorales, ou d'un médicament destiné à traiter des fractures avec défauts osseux, ou d'un médicament destiné à traiter la pseudo-arthrose avec ou sans défaut osseux

L'invention va maintenant être illustrée à titre non limitatif par les exemples suivants :

### Exemple 1 : hydrogel préparé par réticulation de HPMC avec la CMC

Différentes solutions de HPMC E4M (Colorcon, France), ont été préparées à des concentrations en poids de 1%, 1,5%, 2% et 3% du poids total dans de l'eau déminéralisée. Une solution de CMC Blanose 7HCF (Cooper, France) à 2% dans de l'eau déminéralisée a aussi été préparée. Les solutions ont été mélangées pendant 16 heures à température ambiante et dans différentes proportions, versées dans des fioles en verre de 12 ml et fermées avec un bouchon de bromobutyle. Les fioles ont été exposées à une dose de 25 kGy de radiations β dans un réacteur industriel (Ionisos, Chaumesnil, France). Avant irradiation, toutes les solutions étaient visqueuses avec une viscosité comprise entre 1 000 et 16 000 cP. Les différents mélanges obtenus après irradiation se trouvaient sous des formes variées en fonction des proportions de polymères. Les résultats obtenus sont rassemblés dans le Tableau 1.

Les textures des mélanges obtenus étaient très différentes, en fonction des concentrations et proportions en polymères. La présence de CMC a permis d'obtenir un hydrogel homogène dans le cas de l'échantillon 4 (HPMC 1,5 %, CMC 0,5 %) ; alors que pour l'échantillon 2, l'HMPC seule à 1,5 % a conduit à un hydrogel de texture non homogène.

Des analyses infrarouges à transformée de Fourier ont mis en évidence la formation de groupements carbonyles dans le polymère irradié. Ce phénomène est connu pour la cellulose et ses dérivés.

Des mesures de pH ont été effectuées sur l'échantillon 4 : le pH était 5,5 avant stérilisation et atteignait 6,5 après irradiation. Ce changement a été attribué à la transformation des polymères : des radicaux se forment durant l'irradiation et conduisent à la formation de polymères portant des substituants ioniques nouveaux le long de la chaîne cellulosique, ayant un effet sur le pH de la solution.

**Tableau 1 : irradiation des solutions d'HPMC et de CMC (β, 25 kGy)**

| **Echantillons** | **Mélanges polymériques** | | **Résultats** |
|---|---|---|---|
| 1 | **HPMC 1%** | **CMC 2%** | |
| | 100 | 0 | Liquide non visqueux |
| 2 | **HPMC 1,5%** | **CMC 2%** | |
| | 100 | 0 | Hydrogel non homogène |
| | **HPMC 2%** | **CMC 2%** | |
| 3 | 100 | 0 | Hydrogel non homogène |
| 4 | 75 | 25 | Hydrogel homogène |
| 5 | 50 | 50 | Hydrogel non homogène |
| 6 | 25 | 75 | Liquide non visqueux |
| 7 | 0 | 100 | Liquide non visqueux |
| 8 | **HPMC 3%** | **CMC 2%** | |
| | 100 | 0 | Hydrogel homogène |

### Exemple 2 : hydrogel préparé par réticulation de HPMC avec le hyaluronate de sodium

Une solution de HPMC E4M (Colorcon, France), à 2 % en poids dans de l'eau déminéralisée a été préparée. Une solution de hyaluronate de sodium (HA-Na) (Cristalhyal, Soliance, France, viscosité Brookfield : 1 500 cP à 1% en solution) a également été préparée à 2 % en poids dans de l'eau déminéralisée. Les solutions ont été mélangées pendant 24 heures à différentes proportions, versées dans des fioles en verre de 12 ml et fermées avec des bouchons de bromobutyle. Les fioles ont été exposées à une dose de 25 kGy de radiations β dans un réacteur industriel (Ionisos, Chaumesnil, France). Avant irradiation, toutes les solutions étaient visqueuses avec une viscosité comprise entre 1 000 et 16 000 cP. Les mélanges obtenus après irradiation avaient des textures différentes, en fonction des proportions en polymères. Les résultats sont rassemblés dans le Tableau 2.

**Tableau 2 : irradiation des solutions d'HPMC et de HA-Na (β, 25 kGy)**

| **Echantillons** | **HPMC 2%** | **HA-Na 2%** | **Résultats** |
|---|---|---|---|
| 1 | 100 | 0 | hydrogel non homogène |
| 2 | 75 | 25 | hydrogel homogène |
| 3 | 50 | 50 | hydrogel non homogène |
| 4 | 25 | 75 | Liquide non visqueux |
| 5 | 0 | 100 | Liquide non visqueux |

Comme observé pour les mélanges HPMC/CMC, les textures des produits irradiés étaient très différentes en fonction des concentrations et des proportions en polymères. La présence de HA-Na dans le mélange a permis la préparation d'un hydrogel homogène pour la composition suivante : HPMC 1,5 %, HA-Na 0,5 % (m/m).

### Exemple 3 : substitut osseux obtenu à partir d'un hydrogel selon l'invention et de particules de phosphates de calcium

Des granules de BCP ont été incorporées aux polymères hydrosolubles selon l'invention avant irradiation.

Dans le BCP utilisé, le ratio hydroxyapatite/phosphate tricalcique β est de 60/40.

Il a été constaté de façon surprenante que la proportion de granules de BCP et de polymères hydrosolubles était cruciale. Si le mélange contenait une trop grande quantité de polymères, un produit non homogène était obtenu après irradiation (une phase contenant les granules et une autre phase sans granules). A l'opposé, si le mélange de polymères n'était pas présent en quantité suffisante, certains granules n'étaient pas correctement inclus dans l'hydrogel après irradiation.

De plus, et afin d'améliorer la résistance mécanique du substitut osseux à l'intérieur d'une cavité fermée, deux types de granules présentant des tailles différentes ont été utilisés : des granules dont le diamètre est compris entre 80 et 200 µm et des granules de diamètre est compris entre 0,5 et 1 mm. Ainsi, en cas de pression sur le produit, les granules les plus gros jouent le rôle de bouchon pour les granules les plus petits.

Une formule particulière permettant d'obtenir un produit homogène a été sélectionnée après une série de tests. La formulation sélectionnée comprend:
- 45% d'un mélange aqueux d'hydroxypropylméthylcellulose à une concentration en poids de 1,5% et de carboxyméthylcellulose à une concentration en poids de 0,5% ;
- 44% de granules de phosphate de calcium biphasique de diamètre compris entre 80 et 200 µm ; et
- 11% de granules de phosphate de calcium biphasique de diamètre compris entre 0,5 et 1,0 mm.

Le mélange a été préparé sous vide pour obtenir un produit dépourvu de bulles d'air, a été versé directement dans une seringue droite en verre, une canule en inox ou un moule en silicone qui ont été fermés hermétiquement. Le produit a été exposé aux radiations β à une dose de 25 kGy, pour donner un hydrogel incluant les granules de BCP.

Le produit obtenu dans une seringue droite ou une canule peut facilement être extrudé. Si le produit se trouve dans un moule droit, il peut être extrait à la main par simple pression. Par ailleurs, ce nouveau matériau peut être utilisé dans des cavités ouvertes, car l'hydrogel incluant les granules de BCP ne coule pas.

### Exemple 4 : étude du taux de gonflement et du taux de réticulation des hydrogels en fonction de la dose d'irradiations béta

Comme décrit précédemment, selon la dose d'irradiation, le taux de gonflement et le taux de réticulation peuvent être modifiés. Afin de vérifier ce phénomène, une étude portant sur trois mélanges de polymères hydrosolubles a été réalisée. Le taux de gonflement et le taux de réticulation des hydrogels obtenus en fonction de la dose d'irradiations ont été analysés.

Le premier mélange est constitué d'HPMC (E4M, Colorcon) et de CMC (Blanose 7H, Hercules) avec les concentrations en poids respectives de 1,5% et 0,5% en solution dans de l'eau déionisée.

Le second mélange est constitué d'HPMC (E4M, Colorcon) et de hyaluronate de sodium (Crystalhyal, Soliance) avec les concentrations en poids respectives de 1,5% et 0,5% en solution dans de l'eau déionisée.

Le troisième mélange est constitué d'HPMC (E4M, Colorcon) et d'alginate de sodium (Sigma aldrich) avec les concentrations respectives en poids de 1,5% et 0,5% en solution dans de l'eau déionisée.

Les solutions de polymère ont été préparées sous agitation magnétique et ensuite filtrées sur cartouches filtrantes de 5 µm. Les solutions ont été ensuite conditionnées dans des flacons en verre borosilicaté de 20 ml et fermés à l'aide de bouchons bromobutyle. Ces derniers ont eté ensuite exposés à différentes doses d'irradiation beta au centre Ionisos de Chaumesnil (France). Les doses ont été les suivantes : 10, 15, 20, 37,5 et 50 kGy. Pour toutes les doses d'irradiation, il a été observé la formation d'un hydrogel.

Afin de déterminer le taux de gonflement, il a été établi le protocole suivant. Environ 5 g d'hydrogel (m0) sont pesés exactement et écrasés au pilon en présence de 30 ml d'eau déionisée. L'hydrogel en présence d'eau est laissé pendant 24 heures à l'étuve à 37°C dans un bécher fermé par du parafilm. Un témoin est directement séché à 100°C afin de vérifier le pourcentage de polymères secs. Après 24 heures, l'hydrogel est passé sur un papier filtre, il est récupéré et pesé (mh). Le taux de gonflement (Tg) est déterminé par la formule suivante Tg = ((mh-m0)/m0)×100. Pendant la phase de gonflement, la phase polymérique non réticulé se disperse dans l'eau. Afin de déterminer le taux de réticulation, l'hydrogel gonflé est séché à l'étuve 100°C pendant 24 heures. La masse sèche de produit sec est mesurée (ms), le taux de réticulation (Tr) est calculé de la façon suivante : Tr = (ms/(m0×0,02)×100). Les Tableaux ci-dessous présentent les résultats obtenus sur les trois types d'hydrogel.

**Tableau 3 : Taux de gonflement et taux de réticulation pour l'hydrogel (HPMC-CMC) à différentes doses d'irradiations béta.**

| | **10 kGy** | **15 kGy** | **20 kGy** | **25 kGy** | **37,5k Gy** | **50 kGy** |
|---|---|---|---|---|---|---|
| **Tg (%)** | 369 | 261 | 231 | 245 | 236 | 245 |
| **Tr (%)** | 83,3 | 83,3 | 84,5 | 82,5 | 74,0 | 69,1 |

| | | | | | | |
|---|---|---|---|---|---|---|
| **Tableau 4 : Taux de gonflement et taux de réticulation pour l'hydrogel (HPMC-hyaluronate de sodium) à différentes doses d'irradiations béta.** | | | | | | |
| | **10 kGy** | **15 kGy** | **20 kGy** | **25 kGy** | **37,5 kGy** | **50 kGy** |
| **Tg (%)** | 332 | 256 | 202 | 190 | 179 | 167 |
| **Tr (%)** | 78,4 | 82,2 | 82,7 | 78,8 | 71,8 | 67,7 |
| | | | | | | |

| **Tableau 5 : Taux de gonflement et taux de réticulation pour l'hydrogel (HPMC-Alginate de sodium) à différentes doses d'irradiations béta.** | | | | | | |
|---|---|---|---|---|---|---|
| | **10 kGy** | **15 kGy** | **20 kGy** | **25 kGy** | | |
| **Tg (%)** | 280 | 238 | 208 | 185 | | |
| **Tr (%)** | 77,9 | 78,5 | 78,4 | 79,2 | | |

Il est observé que le taux de gonflement diminue avec l'augmentation de la dose d'irradiation pour les trois mélanges. Aussi il apparaît que le taux de réticulation reste inchangé entre 10 et 25 kGy et qu'à partir de 37,5 kGy, le pourcentage de réticulation diminue, ce phénomène se traduisant par un ratio dégradation/réticulation plus important. Il est constaté aussi que le taux de gonflement n'est pas identique selon le polymère associé à l'HPMC : cela s'explique par les propriétés intrinsèques de chaque second polymère. Les mélanges de polymères permettent d'obtenir des taux de gonflement contrôlés en fonction de la dose d'irradiation. Aussi nous avons mis en évidence que le phénomène était reproductible en fonction de la dose d'irradiation.

### Exemple 5 : étude de biocompatibilité des hydrogels et des substituts osseux préparés par irradiation selon l'invention

### 4.1. Produits

Un hydrogel et un substitut osseux ont été préparés selon le Tableau 6. Le BCP était identique à celui utilisé dans les exemples précédents.

L'HPMC était de type E4M (Colorcon, France), la CMC Blanose H7 (Cooper, France), les granules de phosphate de calcium biphasique ont été obtenus auprès de Biomatlante (France). La cytotoxicité a également été testée sur les granules de BCP seuls. Afin de déterminer l'effet de la radiation sur la cytotoxicité du polymère, une fraction des produits a été stérilisée par la vapeur (121°C, 20 minutes) et l'autre par irradiation β (25 kGy).

**Tableau 6 : composition d'hydrogel et de substitut osseux**

| | **Mélange polymérique** | **Particules** |
|---|---|---|
| | 1,5% HPMC + 0,5% CMC | 20% 80-200 µm + 80% 500-1000 µm |
| **Hydrogel** | 100 | 0 |
| **Substitut osseux** | 45 | 55 |

Les compositions des produits testés et leur nomination dans l'étude sont les suivantes :
A Hydrogel, stérilisé par irradiation
B Hydrogel, stérilisé par la vapeur
C Substitut osseux, stérilisé par irradiation
D Substitut osseux, stérilisé par la vapeur
E Granules de BCP, stérilisés par la vapeur

### 4.2. Ligne cellulaire

Des ostéoblastes de souris calvaria (MC3T3-E1) ont été utilisés pour ce test.

### 4.3. Milieu de culture

β-MEM a été utilisé comme milieu de culture. Il a été supplémenté par 1 % de pénicilline/streptomycine, 1 % de L-glutamine et 10 % de sérum de veau foetal (SCF).

### 4.4. Tests

Les échantillons testés ont été soumis à des évaluations de cytotoxicité *in vitro,* d'après la norme de l'International Standard Organization NF EN ISO 10993 : Biological Evaluation of Medical Devices, Part 5 : Tests for *in vitro* cytotoxicity.

La viabilité des cellules a été mesurée par le test MTS (Mitosis Tetrazolium Salt). Cette méthode est fondée sur la capacité des mitochondries à oxyder le sel de tétrazolium dans le formazan. L'intensité de la couleur générée est proportionnelle à l'activité de la déshydrogénase mitochondriale. L'absorbance du formazan a été mesuré à 490 nm, donnant ainsi accès à l'activité cellulaire.

### 4.5. Procédé d'extraction

Des échantillons comprenant 0,2 g d'un produit A-E ont été immergés dans un 1 ml de α-MEM complémenté après la stérilisation. Les échantillons ont été agités vigoureusement pendant 2 minutes toutes les 12 heures et incubés pendant 48 heures à 37°C. Après incubation, les milieux complémentés ont été ultra centrifugés (1 200 tours par minute pendant 8 minutes) et des supernatants ont été mis en contact avec les cellules. Différentes dilutions (1/10 et 1/100) ont été réalisées à partir de ces supernatants (extraits purs).

### 4.6. Contrôles

Deux contrôles ont été utilisés :
- un contrôle positif dans lequel les cellules ont été cultivées sur des puits en plastique avec le α-MEM complémenté.
- un contrôle négatif dans lequel les cellules ont été cultivées sur du plastique avec un milieu complémenté plus 5 µg/ml de D-actinomycine (cytolytique).

### 4.7. Résultats et discussions

Des cellules MC3T3-E1 ont été ensemencées à 10 000 cellules/cm² dans des plaques de 96 puits. A une confluence de 90 %, le milieu de culture a été remplacé par l'extrait pur et ensuite dilué 10 et 100 fois. Parallèlement, les cellules ont été incubées en présence des contrôles positifs et négatifs.

La cytotoxicité a été évaluée en mesurant la viabilité cellulaire avec le test MTS après 24, 48 et 72 heures d'incubation avec les extraits. Les résultats sont présentés dans le Tableau 7. Ils sont exprimés en pourcentage de l'activité MTS du contrôle positif. On considère que la cytotoxicité débute quand l'activité mitochondriale est diminuée de plus de 25 % par rapport au contrôle positif.

Après 24 heures d'incubation, l'activité MTS des cellules cultivées avec les extraits purs n'était pas significativement différente de celle du contrôle positif. L'activité mitochondriale a décru de 14 % pour l'échantillon A, de 15 % pour B, de 4 % pour C, de 0 % pour D et de 3 % pour E. Cela suggère la non toxicité des produits.

**Tableau 7 : Evaluation de la cytotoxicité des hydrogels et substituts osseux.**

| **Echantillons** | **Activité mitochondriale** |
|---|---|
| **Contrôle positif** | 100 |
| **Contrôle négatif** | 10 |
| A | 86 |
| A/10 | 92 |
| A/100 | 98 |
| B | 85 |
| B/10 | 95 |
| B/100 | 100 |
| C | 96 |
| C/10 | 95 |
| C/100 | 100 |
| D | 100 |
| D/10 | 100 |
| D/100 | 92 |
| E | 97 |
| E/10 | 95 |
| E/100 | 92 |

Les cellules en contact avec les extraits purs dilués 10 et 100 fois n'ont pas non plus montré de décroissance significative de l'activité MTS, comparé au contrôle positif. La chute est de 10 % (pour la dilution 10 fois) et de 2 % (pour la dilution 100 fois) pour A, 5 % et 0 % pour B, 5% et 0 % pour C, 0 % et 8 % pour D et 5 % et 8 % pour E.

### 4.8. Conclusion

En conclusion, les extraits des matériaux testés n'ont pas montré de preuve de cytotoxicité (réduction de la viabilité cellulaire de plus de 25 %) pour aucun des produits testés dans les conditions de cette étude. De plus, aucune différence de cytotoxicité n'a été observée entre les produits stérilisés par irradiation et les produits stérilisés à la vapeur, à la fois pour le nouvel hydrogel et le nouveau substitut osseux.

## Revendications

1. Procédé de préparation d'un hydrogel homogène stérile réticulé injectable, **caractérisé en ce qu'**il comprend les étapes successives suivantes :
(a) préparer une solution aqueuse comprenant un polymère hydrosoluble dérivé de la cellulose et au moins un second polymère hydrosoluble, la concentration totale en polymère étant comprise en poids entre 0,5% et 5%, de préférence comprise entre 1 % et 4%, et plus préférentiellement encore comprise entre 1,5% et 3% du poids total, le ratio en poids polymère dérivé de la cellulose/second polymère hydrosoluble étant compris entre 85/15 et 70/30, préférentiellement égal à environ 75/25 ;
(b) éventuellement ajouter des particules solides ;
(c) verser le mélange liquide résultant, et contenant les éventuelles particules solides, dans un récipient et fermer ledit récipient au moyen d'un système imperméable à l'eau et au gaz ;
(d) réticuler le liquide en exposant le récipient contenant le liquide et les éventuelles particules solides à une dose de radiations β ou γ comprise entre 5 et 50 kGy, de préférence comprise entre 20 et 30 kGy, et plus préférentiellement encore environ 25 kGy.

2. Procédé selon la revendication 1, **caractérisé en ce que** le polymère hydrosoluble dérivé de la cellulose est choisi dans le groupe comprenant l'hydroxypropylméthylcellulose, la méthylcellulose, et est de préférence l'hydroxypropylméthylcellulose.

3. Procédé selon l'une quelconque des revendications 1 ou 2, **caractérisé en ce que** le second polymère hydrosoluble est choisi dans le groupe comprenant la carboxyméthylcellulose, les glycosaminoglycanes, la polyvinylpyrolidone, le polyéthylèneglycol, le polyvinylalcool, l'alginate de sodium, et est de préférence la carboxyméthylcellulose ou un glycosaminoglycane.

4. Procédé selon la revendication 3, **caractérisé en ce que** le glycosaminoglycane est choisi dans le groupe comprenant le hyaluronate de sodium, l'acide hyaluronique, le sulfate de dermatan, le sulfate kératine, le sulfate d'héparan, le sulfate de chondroïtine, le chitosan et est de préférence le hyaluronate de sodium.

5. Procédé selon l'une quelconque des revendications précédentes, **caractérisé en ce que** :
- le polymère hydrosoluble dérivé de la cellulose est l'hydroxypropylméthylcellulose à une concentration en poids comprise entre 0,5% et 2,5%, de préférence entre 1% et 2%, et plus préférentiellement encore environ 1,5% du poids total ; et
- le second polymère hydrosoluble est choisi dans le groupe comprenant la carboxyméthylcellulose à une concentration en poids comprise entre 0,1 % et 1 %, de préférence entre 0,25% et 0,7%, et plus préférentiellement encore environ 0,5% du poids total ou le hyaluronate de sodium à une concentration en poids comprise entre 0,1% et 1%, de préférence comprise entre 0,25% et 0,7%, et plus préférentiellement encore environ 0,5% du poids total.

6. Procédé selon l'une quelconque des revendications précédentes, **caractérisé en ce que** la solution aqueuse de polymères comprend en outre au moins un polymère biocompatible.

7. Procédé selon la revendication 6, **caractérisé en ce que** le polymère biocompatible est choisi dans le groupe comprenant l'acide polylactique, les copolymères d'acide polylactique et d'acide polyglycolique, la poly ε caprolactone, les polyhydroxybutyrates, les polyhydroxyvalérates, les copolymères de polyhydroxybutyrates et polyhydroxyvalérates, les polyuréthanes, la cellulose, le polyéthylène, les polycarbonates, les polyméthylméthacrylates, les silicones, les polyamides, l'acide polyglycolique.

8. Procédé selon l'une quelconque des revendications précédentes, **caractérisé en ce que** les particules solides sont choisies dans le groupe comprenant le phosphate de calcium biphasique, le phosphate tricalcique β, l'hydroxyapatite, le sulfate de calcium, le zirconite d'alumine, le nasicon, les bioverres, les vitrocéramiques, les nanoparticules, les composés radio opaques, les mélanges des composés précédents, et est de préférence le phosphate de calcium biphasique dans lequel le ratio hydroxyapatite/phosphate tricalcique β est compris entre 10/90 et 90/10, de préférence compris entre 20/80 et 80/20, et plus préférentiellement encore environ 60/40.

9. Procédé selon l'une quelconque des revendications précédentes, **caractérisé en ce que** la quantité de particules solides est comprise en poids entre 15% et 75%, de préférence comprise entre 30% et 60%, et plus préférentiellement encore environ 55% du poids total.

10. Procédé selon l'une quelconque des revendications précédentes, **caractérisé en ce que** :
- le polymère hydrosoluble dérivé de la cellulose est l'hydroxypropylméthylcellulose ;
- le second polymère hydrosoluble est la carboxyméthylcellulose ou un glycosaminoglycane, le glycosaminoglycane étant de préférence le hyaluronate de sodium ; et
- les particules solides sont des granules de phosphate de calcium biphasique.

11. Procédé selon l'une quelconque des revendications précédentes, **caractérisé en ce que** le mélange aqueux comprend en poids :
- 45% d'un mélange aqueux d'hydroxypropylméthylcellulose et de carboxyméthylcellulose ;
- 44% de granules de phosphate de calcium biphasique de diamètre compris entre 80 et 200 µm dans lequel le ratio hydroxyapatite/phosphate tricalcique β est de 60/40 ; et
- 11 % de granules de phosphate de calcium biphasique de diamètre compris entre 0,5 et 1,0 mm dans lequel le ratio hydroxyapatite/phosphate tricalcique β est de 60/40.

12. Procédé selon l'une quelconque des revendications précédentes, **caractérisé en ce qu'**une substance active est ajoutée après irradiation.

13. Procédé selon la revendication 12, **caractérisé en ce que** la substance active est choisie dans le groupe comprenant les antibiotiques, les facteurs de croissance, les hormones, les peptides, les anti-ostéoporose, les anti-tumoraux.

14. Procédé selon l'une quelconque des revendications précédentes, **caractérisé en ce que** le récipient est une seringue d'injection ou une canule.

15. Procédé selon l'une quelconque des revendications précédentes, **caractérisé en ce qu'**il comprend une étape supplémentaire (e) consistant à déshydrater l'hydrogel obtenu à l'étape (d).

16. Hydrogel susceptible d'être obtenu par la mise en oeuvre d'un procédé selon l'une quelconque des revendications précédentes.

17. Hydrogel selon la revendication 17 pour son utilisation en tant que médicament.

18. Hydrogel selon la revendication 18 en tant que substitut osseux.

## Patentansprüche

1. Verfahren zur Herstellung eines injizierbaren, vernetzten, sterilen, homogenen Hydrogels, **dadurch gekennzeichnet, dass** es die folgenden sukzessiven Schritte umfasst:
(a) Herstellen einer wässrigen Lösung, welche ein von Zellulose abgeleitetes wasserlösliches Polymer und wenigstens ein zweites wasserlösliches Polymer umfasst, wobei die Gesamtkonzentration an Polymer 0,5 Gew.-% bis 5 Gew.-%, bevorzugt 1 Gew.-% bis 4 Gew.-% und noch bevorzugter 1,5 Gew.-% bis 3 Gew.-% des Gesamtgewichts beträgt, wobei das Gewichtsverhältnis von dem von Zellulose abgeleiteten Polymer zu dem zweiten wasserlöslichen Polymer 85/15 bis 70/30, vorzugsweise etwa 75/25, beträgt;
(b) gegebenenfalls Zugabe von Feststoffpartikeln;
(c) Einfüllen der resultierenden und gegebenenfalls Feststoffpartikel enthaltenden flüssigen Mischung in einen Behälter und Schließen des Behälters mit Hilfe eines für Wasser und Gas undurchlässigen Systems;
(d) Vernetzen der Flüssigkeit, indem der Behälter, der die Flüssigkeit und gegebenenfalls die Feststoffpartikel enthält, einer ß- oder γ-Strahlendosis von 5 bis 50 kGy, bevorzugt 20 bis 30 kGy, noch bevorzugter etwa 25 kGy, ausgesetzt wird.

2. Verfahren gemäß Anspruch 1, **dadurch gekennzeichnet, dass** das von Zellulose abgeleitete wasserlösliche Polymer aus der Gruppe umfassend Hydroxypropylmethylzellulose und Methylzellulose ausgewählt ist und vorzugsweise Hydroxypropylmethylzellulose ist.

3. Verfahren gemäß irgendeinem der Ansprüche 1 oder 2, **dadurch gekennzeichnet, dass** das zweite wasserlösliche Polymer aus der Gruppe umfassend Carboxymethylzellulose, Glykosaminoglykane, Polyvinylpyrrolidon, Polyethylenglykol, Polyvinylalkohol und Natriumalginat ausgewählt ist und bevorzugt Carboxymethylzellulose oder ein Glykosaminoglykan ist.

4. Verfahren gemäß Anspruch 3, **dadurch gekennzeichnet, dass** das Glykosaminoglykan aus der Gruppe umfassend Natriumhyaluronat, Hyaloronsäure, Dermatansulfat, Keratinsulfat, Heparansulfat, Chondroitinsulfat und Chitosan ausgewählt ist und bevorzugt Natriumhyaluronat ist.

5. Verfahren gemäß irgendeinem der vorhergehenden Ansprüche, **dadurch gekennzeichnet, dass**:
- das von Zellulose abgeleitete wasserlösliche Polymer Hydroxypropylmethylzellulose mit einer Konzentration von 0,5 Gew.-% bis 2,5 Gew.-%, bevorzugt 1 Gew.-% bis 2 Gew.-% und noch bevorzugter etwa 1,5 Gew.-% des Gesamtgewichts ist; und
- das zweite wasserlösliche Polymer aus der Gruppe umfassend Carboxymethylzellulose mit einer Konzentration von 0,1 Gew.-% bis 1 Gew.-%, bevorzugt 0,25 Gew.-% bis 0,7 Gew.-% und noch bevorzugter etwa 0,5 Gew.-% des Gesamtgewichts, oder Natriumhyaluronat mit einer Konzentration von 0,1 Gew.-% bis 1 Gew.-%, bevorzugt 0,25 Gew.-% bis 0,7 Gew.-% und noch bevorzugter etwa 0,5 Gew.-% des Gesamtgewichts, ausgewählt ist.

6. Verfahren gemäß irgendeinem der vorhergehenden Ansprüche, **dadurch gekennzeichnet, dass** die wässrige Lösung der Polymeren außerdem wenigstens ein biokompatibles Polymer umfasst.

7. Verfahren gemäß Anspruch 6, **dadurch gekennzeichnet, dass** das biokompatible Polymer aus der Gruppe umfassend Polymilchsäure, Polymilchsäure-Polyglykolsäure-Copolymere, Poly-ε-Caprolacton, Polyhydroxybutyrate, Polyhydroxyvalerate, Polyhydroxybutyrat-Polyhydroxyvalerat-Copolymere, Polyurethane, Zellulose, Polyethylen, Polycarbonate, Polymethylmethacrylate, Silicone, Polyamide und Polyglykolsäure ausgewählt ist.

8. Verfahren gemäß irgendeinem der vorhergehenden Ansprüche, **dadurch gekennzeichnet, dass** die Feststoffpartikel aus der Gruppe umfassend biphasisches Calciumphosphat, β-Tricalciumphosphat, Hydroxyapatit, Calciumsulfat, Aluminium-Zirkonit, Nasicon, Bioglas, Vitrokeramik, Nanopartikel, strahlenundurchlässige Verbindungen und Mischungen der zuvor genannten Verbindungen ausgewählt ist und vorzugsweise biphasisches Calciumphosphat ist, bei dem das Verhältnis Hydroxyapatit/β-Tricalciumphosphat 10/90 bis 90/10, bevorzugt 20/80 bis 80/20 und noch bevorzugter etwa 60/40 beträgt.

9. Verfahren gemäß irgendeinem der vorhergehenden Ansprüche, **dadurch gekennzeichnet, dass** die Menge an Feststoffpartikeln 15 Gew.-% bis 75 Gew.-%, bevorzugt 30 Gew.-% bis 60 Gew.-% und noch bevorzugter etwa 55 Gew.-% des Gesamtgewichts beträgt.

10. Verfahren gemäß irgendeinem der vorhergehenden Ansprüche, **dadurch gekennzeichnet, dass**
- das von Zellulose abgeleitete wasserlösliche Polymer Hydroxypropylmethylzellulose ist;
- das zweite wasserlösliche Polymer Carboxymethylzellulose oder ein Glykosaminoglykan ist, wobei das Glykosaminoglykan bevorzugt Natriumhyaluronat ist; und
- die Feststoffpartikel Granulate aus biphasischem Calciumphosphat sind.

11. Verfahren gemäß irgendeinem der vorhergehenden Ansprüche, **dadurch gekennzeichnet, dass** die wässrige Mischung enthält:
- 45 Gew.-% einer wässrigen Mischung aus Hydroxypropylmethylzellulose und Carboxymethylzellulose;
- 44 Gew.-% Granulate aus biphasischem Calciumphosphat mit einem Durchmesser von 80 bis 200 µm, worin das Verhältnis Hydroxyapatit/β-Tricalciumphosphat 60/40 beträgt; und
- 11 Gew.-% Granulate aus biphasischem Calciumphosphat mit einem Durchmesser von 0,5 bis 1 mm, worin das Verhältnis Hydroxyapatit/β-Tricalciumphosphat 60/40 beträgt.

12. Verfahren gemäß irgendeinem der vorhergehenden Ansprüche, **dadurch gekennzeichnet, dass** nach der Bestrahlung ein Wirkstoff zugegeben wird.

13. Verfahren gemäß Anspruch 12, **dadurch gekennzeichnet, dass** der Wirkstoff aus der Gruppe umfassend Antibiotika, Wachstumsfaktoren, Hormone, Peptide, Anti-Osteoporose-Mittel und Anti-Tumor-Mittel ausgewählt ist.

14. Verfahren gemäß irgendeinem der vorhergehenden Ansprüche, **dadurch gekennzeichnet, dass** der Behälter eine Injektionsspritze oder eine Injektionsnadel ist.

15. Verfahren gemäß irgendeinem der vorhergehenden Ansprüche, **dadurch gekennzeichnet, dass** es einen zusätzlichen Schritt (e) umfasst, in dem das in Schritt (d) erhaltene Hydrogel dehydratisiert wird.

16. Hydrogel, welches durch ein Verfahren gemäß irgendeinem der vorhergehenden Ansprüche erhältlich ist.

17. Hydrogel gemäß Anspruch 17 zur Verwendung als Medikament.

18. Hydrogel gemäß Anspruch 18 als Knochenersatz.

## Claims

1. Method for preparing an injectable crosslinked sterile homogenous hydrogel, **characterised in that** it comprises the following successive steps:
(a) preparing an aqueous solution comprising a water-soluble polymer derived from cellulose and at least one second water-soluble polymer, the total polymer concentration being comprised by weight between 0.5% and 5%, preferably comprised between 1% and 4%, and still more preferentially comprised between 1.5% and 3% of the total weight, the weight ratio of polymer derived from cellulose/second water-soluble polymer being comprised between 85/15 and 70/30, preferentially equal to approximately 75/25;
(b) optionally adding solid particles;
(c) pouring the resulting liquid mixture containing the optional solid particles, in a container, and closing said container by means of a system impervious to water and to gas;
(d) crosslinking the liquid by exposing the container containing the liquid and the optional solid particles to a dose of β or γ radiation comprised between 5 and 50 kGy, preferably comprised between 20 and 30 kGy, and still more preferentially approximately 25 kGy.

2. Method according to claim 1, **characterised in that** the water-soluble polymer derived from cellulose is selected from the group comprising hydroxypropyl methylcellulose, methylcellulose and is preferably hydroxypropyl methylcellulose.

3. Method according to any of claims 1 or 2, **characterised in that** the second water-soluble polymer is selected from the group comprising carboxymethylcellulose, glycosaminoglycans, polyvinylpyrrolidone, polyethylene glycol, polyvinyl alcohol, sodium alginate and is preferably carboxymethylcellulose or a glycosaminoglycan.

4. Method according to claim 3, **characterised in that** the glycosaminoglycan is selected from the group comprising sodium hyaluronate, hyaluronic acid, dermatan sulphate, keratin sulphate, heparin sulphate, chondroitin sulphate, chitosan and is preferably sodium hyaluronate.

5. Method according to any of the above claims, **characterised in that**:
- the water-soluble polymer derived from cellulose is hydroxypropyl methylcellulose at a weight concentration comprised between 0.5% and 2.5%, preferably between 1% and 2%, and still more preferentially approximately 1.5% of the total weight; and
- the second water-soluble polymer is selected from the group comprising carboxymethylcellulose at a weight concentration comprised between 0.1% and 1%, preferably between 0.25% and 0.7% and still more preferentially approximately 0.5% of the total weight, or sodium hyaluronate at a weight concentration comprised between 0.1% and 1%, preferably comprised between 0.25% and 0.7% and still more preferentially approximately 0.5% of the total weight.

6. Method according to any of the above claims, **characterised in that** the aqueous solution of polymers further comprises at least one biocompatible polymer.

7. Method according to claim 6, **characterised in that** the biocompatible polymer is selected from the group comprising polylactic acid, copolymers of polylactic acid and of polyglycolic acid, poly(ε-caprolactone), polyhydroxybutyrates, polyhydroxy-valerates, copolymers of polyhydroxybutyrates and polyhydroxyvalerates, polyurethanes, cellulose, polyethylene, polycarbonates, polymethylmethacrylates, silicones, polyamides, polyglycolic acid.

8. Method according to any of the above claims, **characterised in that** the solid particles are selected from the group comprising biphasic calcium phosphate, β tricalcium phosphate, hydroxyapatite, calcium sulphate, alumina zirconite, nasicon, bioglasses, vitroceramics, nanoparticles, radio-opaque compounds, mixtures of the above compounds, and is preferably biphasic calcium phosphate wherein the hydroxyapatite/β tricalcium phosphate ratio is comprised between 10/90 and 90/10, preferably comprised between 20/80 and 80/20, and still more preferentially approximately 60/40.

9. Method according to any of the above claims, **characterised in that** the amount of solid particles is comprised by weight between 15% and 75%, preferably comprised between 30% and 60%, and still more preferentially approximately 55% of the total weight.

10. Method according to any of the above claims, **characterised in that**:
- the water-soluble polymer derived from cellulose is hydroxypropyl methylcellulose;
- the second water-soluble polymer is carboxymethylcellulose or a glycosaminoglycan, the glycosaminoglycan preferably being sodium hyaluronate; and
- the solid particles are biphasic calcium phosphate granules.

11. Method according to any of the above claims, **characterised in that** the aqueous mixture comprises by weight:
- 45% of an aqueous mixture of hydroxypropyl methylcellulose and carboxymethylcellulose;
- 44% of biphasic calcium phosphate granules with a diameter comprised between 80 and 200 µm wherein the hydroxyapatite/β tricalcium phosphate ratio is 60/40; and
- 11% of biphasic calcium phosphate granules with a diameter comprised between 0.5 and 1.0 mm wherein the hydroxyapatite/β tricalcium phosphate ratio is 60/40.

12. Method according to any of the above claims, **characterised in that** an active substance is added after irradiation.

13. Method according to claim 12, **characterised in that** the active substance is selected from the group comprising antibiotics, growth factors, hormones, peptides, anti-osteoporosis agents, anti-tumour agents.

14. Method according to any of the above claims, **characterised in that** the container is an injection syringe or a cannula.

15. Method according to any of the above claims, characterised it comprises an additional step (e) consisting of dehydrating the hydrogel obtained in step (d).

16. Hydrogel obtainable by applying a method according to any of the above claims.

17. Hydrogel according to claim 16 for use thereof as a drug.

18. Hydrogel according to claim 17 as a bone substitute.
